Europäisches Patentamt

European Patent Office

Office européen des brevets

(11) Numéro de publication : **0 447 286 B1**

# (12) FASCICULE DE BREVET EUROPEEN

(45) Date de publication du fascicule du brevet :
01.06.94 Bulletin 94/22

(51) Int. Cl.⁵ : **A61K 7/035, A61K 7/00, A61K 7/48**

(21) Numéro de dépôt : **91400548.3**

(22) Date de dépôt : **28.02.91**

(54) **Composition cosmétique sous forme de poudre compactée contenant des microsphères creuses en matériau synthétique thermoplastique.**

(30) Priorité : **28.02.90 FR 9002509**

(43) Date de publication de la demande :
**18.09.91 Bulletin 91/38**

(45) Mention de la délivrance du brevet :
**01.06.94 Bulletin 94/22**

(84) Etats contractants désignés :
**AT BE CH DE DK ES FR GB GR IT LI NL SE**

(56) Documents cités :
**EP-A- 0 135 060
EP-A- 0 254 612
EP-A- 0 379 409
S.T.N. SERVEUR DE BASES DE DONNEES,
Karsruhe, DE, FICHIER CHEMICAL ABSTRAC-
TS,vol. 109, no. 18, résumé no. 155980n,
Columbus, Ohio, US;**

(56) Documents cités :
**CHEMICAL ABSTRACTS, vol. 104, 1986, page
290, résumé no. 24076w, Columbus,Ohio, US;
& JP-A-60 184 004 (POLA CHEMICAL INDUS-
TRIES, INC.)19-09-1985**

(73) Titulaire : **L'OREAL
14, Rue Royale
F-75008 Paris (FR)**

(72) Inventeur : **Gagnebien, Didier
42, avenue de la Division Leclerc
F-92320 Chatillon (FR)**
Inventeur : **Defossez, Béatrice
87, rue Haxo
F-75020 Paris (FR)**

(74) Mandataire : **Tonnellier, Jean-Claude et al
Cabinet Nony & Cie.
29, rue Cambacérès
F-75008 Paris (FR)**

EP 0 447 286 B1

## Description

L'invention a pour objet l'utilisation, dans la préparation d'une poudre compactée pour le maquillage ou les soins du corps ou du visage, d'un matériau synthétique thermoplastique sous la forme de microsphères creuses de dimensions données, ainsi que des compositions sous forme de poudre compactée contenant un tel matériau.

L'utilisation de microsphères creuses d'un matériau synthétique thermoplastique dans des poudres libres non compactées pour le maquillage et les soins de la peau a déjà été décrite ; voir le brevet FR 86 09289. (n° de publication : 2 600 532).

Cependant, il n'était pas possible d'obtenir des poudres compactées de qualité satisfaisante avec les microsphères de diamètre de 40-60 μm qui étaient utilisées en pratique dans le brevet français cité ci-dessus, en raison de leur élasticité trop importante.

En effet, les poudres compactées doivent présenter des caractéristiques de dureté particulières. La dureté est fonction de la pression de compactage appliquée. Si le produit compact est trop mou, il sera très fragile, et une quantité trop importante de produit sera prélevée au moment de l'application. Par contre, s'il est trop dur, le délitage sera difficile.

Avec les microsphères creuses de 40-60 μm, les essais de compression montraient que la compression était suivie d'un phénomène de relaxation qui provoquait l'apparition, au sein du produit compacté, d'amorces de fragmentation et de morcellement.

Par ailleurs, un produit compact doit présenter une surface parfaitement plane. Enfin, il doit répondre favorablement au test de chute, c'est à dire présenter une perte de poids réduite après une chute dans des conditions précises.

Ces caractéristiques n'étaient pas satisfaites avec les microsphères creuses de 40-60 μm.

Or, on a maintenant découvert qu'en utilisant ce type de matériau synthétique thermoplastique sous forme de microsphères creuses de diamètre moyen inférieur à 30 microns, il est possible d'obtenir des poudres compactées présentant une dureté tout-à-fait satisfaisante, dont l'aspect est correct, et dont la fabrication est aisée.

La présente invention a pour objet l'utilisation, dans la préparation d'une composition cosmétique sous forme de poudre compactée, de microsphères creuses en matériau synthétique thermoplastique ayant une masse spécifique inférieure à 0,1 g/cm³, lesdites microsphères creuses ayant des dimensions inférieures à 30 μm, ladite composition étant exempte d'acyl-lysine.

Les microsphères creuses sort préparées selon des procédés connus, tels que ceux décrits dans le brevet US 3 615 972 et la demande de brevet européen n° 0 056 219.

La partie creuse des microsphères contient un gaz tel qu'un hydrocarbure (butane, pentane), de l'air ou tout autre gaz utilisé classiquement.

Ces microsphères peuvent être réalisées en tous matériaux thermoplastiques non toxiques et non irritants. Ces matériaux peuvent être par exemple des polymères ou copolymères de dérivés éthyléniques (par exemple polyéthylène, polystyrène, copolymère chlorure de vinyle-acrylonitrile, etc...) des polyamides, des polyesters, des polymères urée-formaldéhyde, des copolymères de chlorure de vinylidène (par exemple chlorure de vinylidène-acrylonitrile), etc...

Leurs dimensions doivent être inférieures à 30 μm et sont de préférence de l'ordre de 10 à 20 μm environ.

La masse spécifique des microsphères creuses est de préférence de l'ordre de 0,01 à 0,1 g/cm³.

La proportion pondérale des microsphères creuses dans les poudres compactées varie généralement de 0,02 à 5 % et de préférence de 0,05 à 2 %, par rapport au poids total de la composition.

Les microsphères creuses utilisables dans les compositions de l'invention sont notamment celles qui sont mentionnées dans la partie expérimentale ci-après.

La composition sous forme de poudre compactée contenant les microsphères ainsi définies contient en outre des pigments et/ou des charges.

De préférence, elle contient aussi une phase grasse.

Les pigments et/ou charges sont ceux habituellement utilisés dans les compositions cosmétiques sous forme de poudres compactées. Les pigments sont choisis notamment parmi les pigments minéraux, les pigments organiques et les pigments nacrés.

Parmi les pigments minéraux, on peut citer, à titre d'exemples :
- le dioxyde de titane (rutile ou anatase), éventuellement traité en surface et codifié dans le Color Index sous la référence CI 77891 ;
- les oxydes de fer noir, jaune, rouge et brun, codifiés sous les références CI 77499, 77492, 77491 ;
- le violet de manganèse (CI 77742) ;
- le bleu outremer (CI 77007) ;

- l'oxyde de chrome (CI 77288) ;
- l'oxyde de chrome hydraté (CI 77289) ; et
- le bleu ferrique (CI 77510).

Parmi les pigments organiques, on peut citer, en particulier, les pigments :

D & C red n° 19 (CI 45170) ;
D & C red n° 9 (CI 15585) ;
D & C red n° 21 (CI 45380) ;
D & C orange n° 4 (CI 15510) ;
D & C orange n° 5 (CI 45370) ;
D & C red n° 27 (CI 45410) ;
D & C red n° 13 (CI 15630) ;
D & C red n° 7 (CI 15 850:1) ;
D & C red n° 6 (CI 15 850:2) ;
D & C yellow n° 5 (CI 19140) ;
D & C red n° 36 (CI 12085) ;
D & C orange n°10 (CI 45425) ;
D & C yellow n° 6 (CI 15985) ;
D & C red n° 30 (CI 73360) ;
D & C red n° 3 (CI 45430) ;
le noir de carbone (CI 77266) ; et
les laques à base de carmin de cochenille (CI 75470).

Les pigments nacrés peuvent être choisis notamment parmi :

- les pigments nacrés blancs, tels que le mica recouvert d'oxyde de titane, l'oxychlorure de bismuth ; et
- les pigments nacrés coloris, tel que le mica titane avec des oxydes de fer, le mica titane avec du bleu ferrique ou de l'oxyde de chrome, le mica titane avec un pigment organique du type précité, ainsi que ceux à base d'oxychlorure de bismuth.

Ces pigments peuvent représenter jusqu'à 70 % du poids total de la composition.

Les charges sont choisies notamment parmi :

- Le talc, qui est un silicate de magnésium hydraté, utilisé sous forme de particules généralement de dimensions inférieures à 40 μm ; le talc possède des propriétés absorbantes de l'humidité et est utilisé surtout en raison de son toucher onctueux ;
- les micas, qui sont des aluminosilicates de compositions variées, qui se présentent sous la forme d'écailles ayant des dimensions de 2 à 200 μm, de préférence de 5 à 70 μm et une épaisseur de 0,1 à 5 μm, de préférence de 0,2 à 3 μm. Les micas peuvent être d'origine naturelle (par exemple muscovite, margarite, roscoelithe, lipidolithe, biotite) ou d'origine synthétique. Les micas sont généralement transparents et permettent de conférer à la peau un aspect satiné ;
- l'amidon, en particulier l'amidon de riz ;
- le kaolin, qui est un silicate d'aluminium hydraté, qui se présente sous la forme de particules de forme isotrope ayant des dimensions généralement inférieures à 30 μm, et qui possède de bonnes propriétés d'absorption des corps gras ;
- les oxydes de zinc et de titane, généralement utilisés sous la forme de particules ayant des dimensions ne dépassant pas quelques micromètres (ou même inférieures à 1 μm dans le cas de l'oxyde de titane) ; ces oxydes ont un toucher onctueux, ont un bon pouvoir couvrant et ont une opacité importante ;
- le carbonate de calcium précipité qui, sous forme de particules de dimensions inférieures à 10 μm, a un toucher onctueux et permet d'obtenir un aspect mat ;
- le carbonate ou l'hydrocarbonate de magnésium, qui possèdent notamment des propriétés de fixation des parfums ;
- des savons métalliques dérivés d'acide organique carboxylique ayant de 8 à 22 atomes de carbone, de préférence de 12 à 18 atomes de carbone, par exemple le stéarate de zinc, de magnésium ou de lithium, le laurate de zinc, le myristate de magnésium, etc... Ces savons, présents généralement sous la forme de particules ayant des dimensions inférieures à 10 μm, ont un toucher onctueux et facilitent l'adhérence de la poudre sur la peau ;
- les poudres de polymères synthétiques, tels que le polyéthylène, les polyesters (par exemple isophtalate ou téréphtalate de polyéthylène), et les polyamides (par exemple le nylon), sous la forme de particules ayant des dimensions inférieures à 50 μm, qui possèdent des propriétés absorbantes et permettent de conférer à la peau un aspect velouté.

Ces charges peuvent représenter jusqu'à 95 % du poids total de la composition.

Pigments et charges peuvent être enrobés par des substances telles que des acides aminés, des silicones,

3

des sels métalliques ou du collagène, notamment afin de modifier leur état de surface.

La phase grasse est constituée d'au moins un corps gras, liquide ou solide à température ambiante, et/ou d'un polymère synthétique oléosoluble dont l'utilisation dans les cosmétiques est connue.

Parmi les corps gras liquides à température ambiante, on peut citer les huiles minérales, animales, végétales ou synthétiques, ou encore les huiles ou cires liquides de silicone. Il s'agit par exemple des huiles suivantes : l'huile de vaseline, la lanoline liquide, l'huile d'arara, les huiles de sésame, de macadamia ou de jojoba, et les triglycérides de synthèse.

Parmi les polymères synthétiques oléosolubles, on peut citer les copolymères polyvinylpyrrolidone/hexadécène ou PVP/eicosène tels que les produits vendus par GAF Corp. sous la marque commerciale GANEX V-216 et GANEX V-220.

La phase grasse éventuellement ajoutée représente 0 à 25 % du poids total de la composition, et de préférence 3 à 20 %.

D'autres ingrédients divers peuvent être introduits dans les compositions à compacter, tels que des antiseptiques (trichloro diphényl éther, agents cationiques, acide borique, etc...) qui sont utilisés notamment dans les poudres désodorisantes pour le corps ou les pieds et dans les poudres pour bébés ; des agents astringents, qui sont utilisés dans les poudres désodorisantes ou dans les poudres pour les pieds, tels que l'hydroxychlorure d'aluminium ou les aluns ; des filtres solaires ; des agents adoucissants ; des agents hydratants (sorbitol, glycérine) ; des agents cicatrisants ; des agents anti-radicaux libres ; des vitamines ; des parfums ; etc...

Ces ingrédients peuvent représenter jusqu'à 5 % du poids total de la composition.

Enfin, les poudres compactées selon l'invention contiennent éventuellement des agents de consistance hydrosolubles (jusqu'à 20 % en poids de la solution aqueuse), tels que des gommes naturelles ou synthétiques, des dérivés de cellulose ou des polymères acryliques.

Les poudres de l'invention sont préparées selon les techniques classiques de préparation des poudres compactées. Généralement, on opère de la façon suivante :

Les pigments et/ou charges, ainsi que les additifs poudreux éventuellement présents sont mélangés aux microsphères creuses.

La phase grasse et/ou les agents de consistance, ainsi que les éventuels autres ingrédients sont ajoutés, et le tout est mélangé.

Le mélange résultant est alors compacté à l'aide d'une presse classique ou d'une compacteuse automatique dans des coupelles métalliques.

ESSAIS COMPARATIFS

Des études de l'évolution des duretés de poudres compactées en fonction de la pression de compactage ont été effectuées. Ces études ont comparé plusieurs poudres contenant des microsphères de granulométries différentes, et aussi des quantités d'huiles différentes. Les microsphères utilisées sont les produits commercialisés sous les dénominations EXPANCEL 551 DE (40 μm), 551 DE 20 (20 μm) et 551 DE 12 (12 μm) par la Société KEMANORD PLAST.

Les compositions étudiées sont les suivantes (tableau 1) :

EP 0 447 286 B1

TABLEAU 1

| FORMULES | 1 | 2 | 3 | 1' | 2' |
|---|---|---|---|---|---|
| Oxyde de chrome | 15 | 15 | 15 | 15 | 15 |
| Mica titane | 35 | 35 | 35 | 35 | 35 |
| Talc | 40,8 | 40,8 | 40,8 | 29,5 | 29,5 |
| Huile de vaseline | 5,4 | 5,4 | 5,4 | 20 | 20 |
| Lanoline | 0,5 | 0,5 | 0,5 | – | – |
| Alcool oléique | 1 | 1 - | 1 | – | – |
| Vaseline | 1 | 1 | 1 | – | – |
| Myristate d'isopropyle | 0,8 | 0,8 | 0,8 | – | – |
| Parahydroxybenzoate de propyle | 0,2 | 0,2 | 0,2 | 0,2 | 0,2 |
| Expancel 551 DE | 0,3 | – | – | 0,3 | – |
| Expancel 551 DE 20 | – | 0,3 | – | – | 0,3 |
| Expancel 551 DE 12 | – | – | 0,3 | – | – |

RESULTATS

- Dureté : elle est mesurée par indentation à l'aide d'un duromètre de la marque "ZWICK", référence 3114. Pour ce genre de compositions, la dureté optimale se situe aux environs de 40°Shore A. Cette dureté est atteinte pour des pressions de compactage de 70 bars environ pour la composition 2 et de 55 bars environ pour la composition 3. Par contre, pour la composition 1, la dureté n'atteint 35°Shore A que pour une pression de compactage de 100 bars environ, et n'atteint pas encore 40°Shore A pour une pression de compactage de 120 bars.

  Pour la composition 2', la dureté de 40°Shore A est atteinte pour une pression de 60 bars environ, alors qu'avec la composition 1', la dureté n'atteint pas 35°Shore A pour une pression de 100 bars.

- Test de chute : Ce test consiste à lâcher la poudre compactée à deux reprises d'une hauteur de 1 mètre, puis à déterminer par pesée le pourcentage de poudre éliminée. Dans ce test, la norme d'acceptation est une perte de poids inférieure à 10 %.

  Pour les compositions 2 et 3, la perte de poids est inférieure à 10 % pour des pressions de compactage supérieures à 75 et à 60 bars environ, respectivement, et la perte de poids devient nulle pour des pressions de compactage supérieures à 80 bars. Pour la composition 1, la perte de poids reste supérieure à 30 %, même pour des pressions de compactage atteignant 120 bars.

  Pour la composition 2', la perte de poids est inférieure à 10 % pour des pressions de compactage supérieures à 55 bars environ, et devient nulle pour des pressions supérieures ou égales à 80 bars. Pour la composition 1', la perte de poids et encore supérieure à 20 % pour une pression de compactage de 100 bars.

- Etat de surface : pour une pression de compactage de 80 bars, l'état de surface des compositions 2 et 3 est satisfaisant. Pour la composition 1, la surface n'est pas plane et on observe des pertes de matière au voisinage des bords de la coupelle.

EXEMPLES

Dans les exemples qui suivent, les compositions ont été préparées de la façon décrite précédemment. Dans ces exemples, les compositions sont définies par la teneur pondérale des ingrédients.

5

EXEMPLE 1 : FARD A JOUES

```
Dioxyde de titane................................    10
Mica titane coloré..............................    30
D & C red 30....................................     1,2
Parahydroxybenzoate de propyle..................     0,2
Huile de vaseline...............................    6
Expancel 551 DE 12..............................     0,8
Hydroxyméthoxybenzophénone tel que le produit UVINUL
M40 vendu par la société BASF...................     0,5
Talc...............................q.s.p........   100
```

On applique ce fard à joues à l'aide d'un pinceau.

EXEMPLE 2 : POUDRE COMPACTEE POUR LE VISAGE

```
Poudre de polyéthylène..........................    5
Oxydes de fer...................................    6
Dioxyde de titane...............................   10
Mica............................................   15
Myristate d'isopropyle..........................    1,5
Huile de vaseline...............................    1,5
Expancel 551 DE 20..............................    0,5
Sorbitol........................................    0,5
Talc...........................  q.s.p.......   100
```

On applique cette poudre à l'aide d'une houppette ou d'un pinceau.

EXEMLE 3 : POUDRE COMPACTEE POUR LE VISAGE

```
Dioxyde de titane...............................   10
Amidon de riz...................................   15
Oxydes de fer...................................    8
Cyclométhicone .................................    5
Parahydroxybenzoate de propyle..................    0,2
Poudre de polyamide.............................   10
Expancel 551 DE 12..............................    1,5


Vitamine E......................................    0,5
Talc enrobé par des silicones............q.s.p...   100
```

On applique cette poudre à l'aide d'une houppette ou d'un pinceau.

Dans la formulation précédente, on peut remplacer la cyclométhicone par la même quantité de polydiméthylsiloxane.

EXEMPLE 4 : POUDRE PARFUMEE CORPORELLE (compactée)

```
Oxyde de fer................................   0,07
Stéarate de zinc.............................   6
Parahydroxybenzoate de propyle...................   0,2
Expancel 551 DE 20..........................   0,5
Parfum...................................   4
Talc...........................q.s.p....   100
```

On applique cette poudre à l'aide d'une houppette ou d'un pinceau.

EXEMPLE 5 : FOND DE TEINT A SEC OU A L'EAU

"Two-way cake"

```
Oxychlorure de bismuth.......................   15
Amidon....................................   10
Oxydes de fer.............................   6
Dioxyde de titane.........................   7
Huile de vaseline.........................   4
Expancel 551 DE 12........................   1,2
Gomme arabique (solution aqueuse à 30 %)..........   3
Talc..........................q.s.p.....   100
```

Ce fond de teint peut être utilisé soit à sec, c'est-a-dire à l'aide d'une houppette ou d'un pinceau, soit à l'eau, c'est-à-dire à l'aide d'une éponge (en latex) préalablement humidifiée.

EXEMPLE 6 : FARD A PAUPIERES

```
Oxyde de chrome..........................   6
Oxyde de fer............................   7
Poudre de polyamide......................   15
Cyclométhicone..........................   9
Mica titane.............................   30
Expancel 551 DE 12......................   0,9
Talc..........................q.s.p....   100
```

On applique ce fard à paupières à l'aide d'un applicateur en mousse ou en latex ou à l'aide d'un pinceau.

Dans la formulation précédente, on peut remplacer la cyclométhicone par la même quantité d'hexadécyldiméthicone.

EXEMPLE 7 : POUDRE COMPACTEE POUR LE VISAGE

```
Poudre de polyamide ...........................   10

Mica.........................................   30

Stéarate de zinc.............................    4

Dioxyde de titane............................    2

Copolymère polyvinylpyrrolidone/hexadécène

(GANEX V-216 vendu par la société GAF)..........  0,5

Huile de vaseline............................   5,5

Oxyde de fer.................................    3

Expancel 551 DE 20...........................   0,3

Talc...............................q.s.p......  100
```

On applique cette poudre à l'aide d'une houppette ou d'un pinceau.

**Revendications**

1. Utilisation dans la préparation d'une composition cosmétique sous forme de poudre compactée, de microsphères creuses, en matériau synthétique thermoplastique, ayant une masse spécifique de 0,01 à 0,1g/cm³, lesdites microsphères creuses ayant des dimensions inférieures à 30 μm, et ladite composition étant exempte d'acyl-lysine.

2. Utilisation selon la revendication 1, caractérisée par le fait que les dimensions des microsphères creuses sont de l'ordre de 10 à 20 μm.

3. Utilisation selon l'une quelconque des revendications précédentes, caractérisée par le fait que ledit matériau thermoplastique est choisi parmi les polymères ou copolymères dérivés d'hydrocarbures éthyléniques, des polyesters, des polyamides, des polymères urée-formaldéhydes et des copolymères de chlorure de vinylidène.

4. Utilisation selon la revendication 3, caractérisée par le fait que ledit matériau est un copolymère chlorure de vinylidène-acrylonitrile.

5. Utilisation selon l'une quelconque des revendications précédentes, caractérisée par le fait que lesdites microsphères creuses sont présentes dans des proportions pouvant aller de 0,02 à 5 % en poids, et en particulier de 0,05 à 2 % en poids par rapport au poids total de la composition.

6. Utilisation selon l'une quelconque des revendications précédentes, caractérisée par le fait que lesdites microsphères creuses contiennent un gaz.

7. Composition cosmétique sous forme de poudre compactée, contenant des charges et/ou des pigments usuels, caractérisée par le fait qu'elle contient en outre des microsphères creuses, en matériau synthétique thermoplastique, ayant une masse spécifique de 0,01 à 0,1g/cm³, et des dimensions inférieures à 30 μm et ladite composition étant exempte d'acyl-lysine.

8. Composition selon la revendication 7, caractérisée par le fait que lesdites microsphères creuses sont telles que définies dans l'une quelconque des revendications 2 à 4.

9. Composition selon l'une quelconque des revendications 7 et 8, caractérisée par le fait qu'elle contient de 0,02 à 5 %, et en particulier de 0,05 à 2 % en poids de microsphères creuses, par rapport au poids total de la composition.

10. Composition selon l'une quelconque des revendications 7 à 9, caractérisée par le fait que lesdites microsphères creuses contiennent un gaz.

11. Composition selon l'une quelconque des revendications 7 à 10, caractérisée par le fait que lesdites charges sont présentes dans des proportions pouvant aller jusqu'à 95 % en poids du poids total de la composition.

12. Composition selon l'une quelconque des revendications 7 à 11, caractérisée par le fait qu'elle contient en outre 0 à 25 % en poids d'une phase grasse, par rapport au poids total de la composition.

**Patentansprüche**

1. Verwendung von Mikrohohlkügelchen aus synthetischem thermoplastischen Material mit einer spezifischen Dichte von 0,01 bis 0,1 g/cm³ bei der Herstellung einer kosmetischen Zubereitung in Form eines Kompaktpuders, wobei die Mikrohohlkügelchen eine Größe von kleiner als 30 μm haben und die Zubereitung frei von Acyllysin ist.

2. Verwendung gemäß Anspruch 1, dadurch gekennzeichnet, daß die Größe der Mikrohohlkügelchen in dem Bereich von 10 bis 20 μm liegt.

3. Verwendung gemäß irgendeinem der vorstehenden Ansprüche, dadurch gekennzeichnet, daß das thermoplastische Material aus von Ethylenkohlenwasserstoffen abgeleiteten Polymeren oder Copolymeren, Polyestern, Polyamiden, Harnstoff-Formaldehyd-Polymeren und Vinylchlorid-Copolymeren ausgewählt ist.

4. Verwendung gemäß Anspruch 3, dadurch gekennzeichnet, daß das Material ein Vinylchlorid-Acrylnitril-Copolymer ist.

5. Verwendung gemäß irgendeinem der vorstehenden Ansprüche, dadurch gekennzeichnet, daß die Mikrohohlkügelchen in Anteilen, die von 0,02 bis 5 Gew.-% und insbesondere von 0,05 bis 2 Gew.-%, bezogen auf das Gesamtgewicht der Zubereitung, variiren können, vorliegen.

6. Verwendung gemäß irgendeinem der vorstehenden Ansprüche, dadurch gekennzeichnet, daß die Mikrohohlkügelchen ein Gas enthalten.

7. Kosmetische Zubereitung, in Form eines Kompaktpuders und einem Gehalt an den üblichen Zusätzen und/oder Pigmenten enthält, dadurch gekennzeichnet, daß sie darüber hinaus Mikrohohlkügelchen aus einem synthetischen thermoplastischen Material mit einer spezifischen Dichte von 0,01 bis 0,1 g/cm³ und einer Größe von kleiner als 30 μm enthält und die Zubereitung frei von Acyllysin ist.

8. Zubereitung gemäß Anspruch 7, dadurch gekennzeichnet, daß die Mikrohohlkügelchen in einer nach einem der Ansprüche 2 bis 4 definierten Form vorliegen.

9. Zubereitung gemäß einem der Ansprüche 7 und 8, dadurch gekennzeichnet, daß sie 0,02 bis 5 und insbesondere 0,05 bis 2 Gew.-% Mikrohohlkügelchen, bezogen auf das Gesamtgewicht der Zubereitung, enthält.

10. Zubereitung gemäß irgendeinem der Ansprüche 7 bis 9, dadurch gekennzeichnet, daß die Mikrohohlkügelchen ein Gas enthalten.

11. Zubereitung gemäß einem der Ansprüche 7 bis 10, dadurch gekennzeichnet, daß die Zusätze in Anteilen vorliegen, die bis zu 95 Gew.-% des Gesamtgewichts der Zubereitung ausmachen können.

12. Zubereitung gemäß einem der Ansprüche 7 bis 11, dadurch gekennzeichnet, daß sie außerdem 0 bis 25 Gew.-% einer Fettphase, bezogen auf das Gesamtgewicht der Zubereitung, enthält.

## Claims

1.  Use in the preparation of a cosmetic composition in the form of compacted powder, of hollow microspheres, made from thermoplastic synthetic material, having a density of 0.01 to 0.1 g/cm$^3$, the said hollow microspheres being of sizes of less than 30 μm, and the said composition being free of acyl lysine.

2.  Use according to Claim 1, characterized by the fact that the sizes of the hollow microspheres are of the order of 10 to 20 μm.

3.  Use according to any one of the preceding claims, characterized by the fact that the said thermoplastic material is chosen from polymers or copolymers derived from ethylene hydrocarbons, polyesters, polyamides, urea-formaldehyde polymers and vinylidene chloride copolymers.

4.  Use according to Claim 3, characterized by the fact that the said material is a vinylidene chloride-acrylonitrile copolymer.

5.  Use according to any one of the preceding claims, characterized by the fact that the said hollow microspheres are present in proportions which may range from 0.02 to 5 % by weight, and in particular from 0.05 to 2 % by weight relative to the total weight of the composition.

6.  Use according to any one of the preceding claims, characterized by the fact that the said hollow microspheres contain a gas.

7.  Cosmetic composition in the form of compacted powder, containing customary fillers and/or pigments, characterized by the fact that it contains, in addition, hollow microspheres, made from thermoplastic synthetic material, having a density of 0.01 to 0.1 g/cm$^3$, and of sizes of less than 30 μm, and the said composition being free of acyl lysine.

8.  Composition according to Claim 7, characterized by the fact that the said hollow microspheres are as defined in any one of Claims 2 to 4.

9.  Composition according to any one of Claims 7 and 8, characterized by the fact that it contains from 0.02 to 5 % and in particular from 0.05 to 2 % by weight of hollow microspheres, relative to the total weight of the composition.

10. Composition according to any one of Claims 7 to 9, characterized by the fact that the said hollow microspheres contain a gas.

11. Composition according to any one of Claims 7 to 10, characterized by the fact that the said fillers are present in proportions which may range up to 95 % by weight of the total weight of the composition.

12. Composition according to any one of Claims 7 to 11, characterized by the fact that it contains, in addition, 0 to 25 % by weight of a fatty phase, relative to the total weight of the composition.